# EUROPEAN PATENT APPLICATION

(11) **EP 4 414 368 A1**
(43) Date of publication of application: **14.08.2024**
(21) Application number: 22878884.0
(22) Date of filing: 05.10.2022
(51) Int. Cl.: C07D 493/04, H01M 10/0567, H01M 10/052

(54) **SATURATED FURODIOXIN DERIVATIVE COMPOUND AND USE THEREOF AS ADDITIVE FOR SECONDARY BATTERY**

(30) Priority: 06.10.2021 KR 20210132604
(71) Applicant: Samyang Corporation, Jongno-gu Seoul 03129 (KR)
(72) Inventor: AHN, Jung Min, Busan 48056 (KR); LEE, Chi Wan, Daejeon 34127 (KR); LEE, Kwang Hee, Seoul 06372 (KR); LEE, Jae Hoon, Daejeon 34049 (KR); IM, Jun Seop, Hwaseong-si Gyeonggi-do 18496 (KR); JEE, Chang Do, Seoul 06586 (KR)
(74) Representative: Clarenbach, Carl-Philipp
(86) International application number: PCT/KR2022/014989
(87) International publication number: WO 2023/059063

(57) **Abstract**

The present invention pertains to a saturated furodioxin derivative compound, and a use thereof as an additive for a secondary battery. More specifically, the present invention pertains to a novel compound and a method for preparing same, an electrolyte additive and a non-aqueous electrolyte composition containing the compound, and a secondary battery (in particular, a lithium-sulfur secondary battery) including the electrolyte composition, wherein the compound has a structure in which a substituent is bonded to a saturated furodioxin backbone. When the compound is contained as an additive in a non-aqueous electrolyte composition for a secondary battery (in a non-aqueous electrolyte composition for a lithium-sulfur secondary battery), the problem that conventional electrolyte compositions have of lithium polysulfide dissolution can be resolved, and high capacity retention rate characteristics can be achieved for various lithium salts.

## Description

### TECHNICAL FIELD

The present invention relates to a saturated furodioxine derivative compound and use thereof as additive for secondary battery, and more specifically, the present invention relates to a novel compound having a saturated furodioxine backbone structure with substituent bonded thereto, which can, if contained as additive in non-aqueous electrolyte composition for secondary battery (in particular, non-aqueous electrolyte composition for lithium-sulfur secondary battery), solve the problem of lithium polysulfide elution in conventional electrolyte compositions and simultaneously achieve high capacity retention rate characteristics for various lithium salts, and a preparation method thereof, and an electrolyte additive and a non-aqueous electrolyte composition comprising the compound, and a secondary battery (in particular, a lithium-sulfur secondary battery) comprising the electrolyte composition.

### BACKGROUND ART

Recently, according to the development of portable electronic devices, electric vehicles and large-capacity power storage systems, etc., the need for large-capacity batteries is emerging. Lithium-sulfur secondary battery is a secondary battery using a sulfur-based material with an S-S bond (sulfur-sulfur bond) as cathode active material and lithium metal as anode active material, and as the main material of the cathode active material, sulfur has the advantage of being very highly resource-rich, non-toxic, and having a low weight per atom.

In addition, the theoretical discharge capacity and theoretical energy density of lithium-sulfur secondary battery are 1,672 mAh/g-sulfur and 2,600 Wh/kg, respectively, which is very high as compared with the theoretical energy densities of other battery systems currently being studied (Ni-MH battery: 450 Wh/kg, Li-FeS battery: 480 Wh/kg, Li-MnO₂ battery: 1,000 Wh/kg, Na-S battery: 800 Wh/kg), and thus lithium-sulfur secondary battery is attracting attention as a battery with high energy density characteristics.

The problem to be solved with most priority for commercialization of lithium-sulfur secondary battery is the low life of the battery due to lithium polysulfide elution. Lithium polysulfide (Li₂Sₓ, x = 8, 6, 4 or 2) is an intermediate product generated during the electrochemical reaction of a lithium-sulfur secondary battery, and has high solubility in organic liquid electrolyte. Lithium polysulfide dissolved in liquid electrolyte gradually diffuses toward the anode and escapes the electrochemical reaction area of the cathode, and so it cannot participate in the electrochemical reaction of the cathode, eventually causing capacity loss. In addition, elution of lithium polysulfide increases the viscosity of the liquid electrolyte and so decreases ionic conductivity, and during continuous charge/discharge reactions, lithium polysulfide reacts with the anode made of a lithium metal material, and so lithium sulfide (Li₂S) is fixed to the surface of the lithium metal, thereby lowering reaction activity and degrading potential characteristics.

Most of the studies to solve such problems are focused on modification of the cathode. Concretely, as one of the ways to increase the electrical conductivity of electrodes, attempts are being made to minimize the conductivity drop of electrode with accumulated lithium sulfide by adding a conductive material made of carbon material, or to control the generation and accumulation of intermediate products and lithium sulfide by using a sulfur carrier having a nanostructure (for example, Korean Patent Nos. 10-1481234 and 10-2244915).

However, the conventional technologies as above are difficult to apply commercially, and can be used up to only 70% of their theoretical capacity. Therefore, there is still a need to develop a lithium-sulfur secondary battery that can delay passivation of electrode due to lithium sulfide and secure high discharge capacity and life characteristics.

### PROBLEMS TO BE SOLVED

The purpose of the present invention is to provide a novel compound which can, if contained as additive in non-aqueous electrolyte composition for secondary battery (in particular, non-aqueous electrolyte composition for lithium-sulfur secondary battery), solve the problem of lithium polysulfide elution in conventional non-aqueous electrolyte, and simultaneously, can enhance the stability of the lithium electrode and achieve high capacity retention rate characteristics, and a preparation method thereof, and an electrolyte additive and a non-aqueous electrolyte composition comprising the compound, and a secondary battery (in particular, a lithium-sulfur secondary battery) comprising the electrolyte composition.

### TECHNICAL MEANS

The present invention provides a compound represented by the following formula 1: 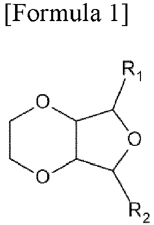 wherein R₁ and R₂ are each independently selected from the group consisting of hydrogen atom, halogen atom, substituted or unsubstituted C1-C30 alkyl group, substituted or unsubstituted C2-C30 alkenyl group, substituted or unsubstituted C2-C30 alkynyl group, substituted or unsubstituted C1-C30 alkoxy group, substituted or unsubstituted C3-C30 cycloalkyl group, substituted or unsubstituted C1-C30 alkylene-O-C1-C30 alkyl group, substituted or unsubstituted C3-C30 heterocycloalkyl group, substituted or unsubstituted C6-C60 aryl group, substituted or unsubstituted C1-C60 heteroaryl group, substituted or unsubstituted C7-C60 aralkyl group, substituted or unsubstituted C6-C60 aryloxy group, and substituted or unsubstituted C6-C60 arylthio group, provided that at least one of R₁ and R₂ is not hydrogen atom.

In an embodiment of the present invention, the compound represented by the above formula 1 may be selected from the group consisting of compounds represented by the following formulas 2 to 4: 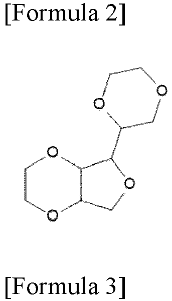 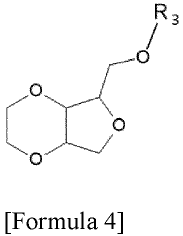 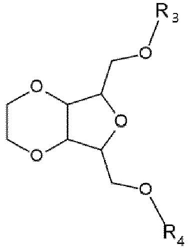 wherein R₃ and R₄ are each independently substituted or unsubstituted C1-C30 alkyl group.

More concretely, the compound represented by the above formula 2 may be selected from the group consisting of compounds represented by the following formulas 2-1 to 2-3; the compound represented by the above formula 3 may be a compound represented by the following formula 3-1 or 3-2; and the compound represented by the above formula 4 may be a compound represented by the following formula 4-1 or 4-2:

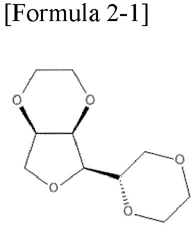

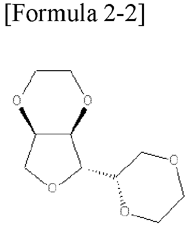

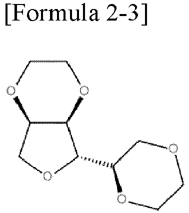

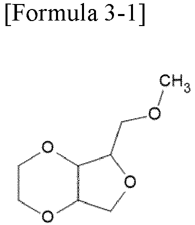

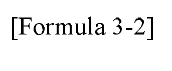

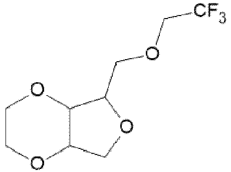

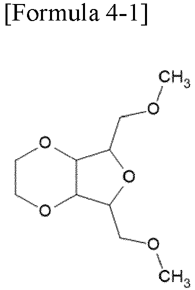

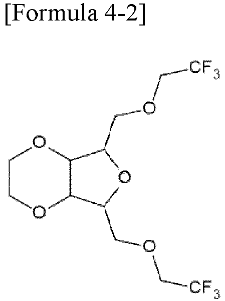

The other aspect of the present invention provides a method for preparing a compound represented by the above formula 2, the method comprising a step of heating dianhydrosugar hexitol-ethylene glycol, which is a compound in which ethylene glycol is added to the hydroxy groups at both ends of dianhydrosugar hexitol, in the presence of an acid catalyst.

Another aspect of the present invention provides a method for preparing a compound represented by the above formula 3 or 4, the method comprising a step of heating a compound represented by the following formula A or B, respectively, and an alkylating agent in the presence of a base catalyst:

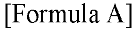

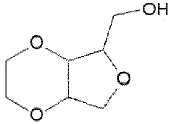

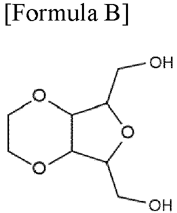

Still another aspect of the present invention provides an electrolyte additive comprising the compound represented by the above formula 1.

Still another aspect of the present invention provides a non-aqueous electrolyte composition comprising: a non-aqueous electrolyte solvent; lithium salt; and the electrolyte additive of the present invention, wherein the amount of the electrolyte additive is from 0.1 to 10 parts by weight, based on total 100 parts by weight of the electrolyte composition.

Still another aspect of the present invention provides a secondary battery comprising: a cathode; an anode; a separation membrane; and the non-aqueous electrolyte composition of the present invention.

### EFFECT OF THE INVENTION

If the compound of formula 1 according to the present invention is used as an electrolyte additive in a non-aqueous electrolyte composition for secondary battery (in particular, non-aqueous electrolyte composition for lithium-sulfur secondary battery), it is possible to provide a non-aqueous electrolyte composition which can solve the problem of lithium polysulfide elution as compared with conventional non-aqueous electrolyte composition, and simultaneously can enhance the stability of the lithium electrode and the capacity retention rate over cycle, and thus can improve battery life characteristics, and a secondary battery (in particular, a lithium-sulfur secondary battery) comprising same.

In addition, according to an embodiment of the present invention, because the compound of formula 1 can be prepared from dianhydrosugar hexitol derived from natural resources, by using it as an additive for secondary batteries, eco-friendliness in the secondary battery field can be improved.

### CONCRETE MODE FOR CARRYING OUT THE INVENTION

The present invention is explained in more detail below.

### [Compound of formula 1 and preparation method thereof]

An aspect of the present invention relates to a compound represented by the following formula 1: 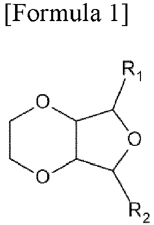 wherein R₁ and R₂ are each independently selected from the group consisting of hydrogen atom, halogen atom, substituted or unsubstituted C1-C30 alkyl group, substituted or unsubstituted C2-C30 alkenyl group, substituted or unsubstituted C2-C30 alkynyl group, substituted or unsubstituted C1-C30 alkoxy group, substituted or unsubstituted C3-C30 cycloalkyl group, substituted or unsubstituted C1-C30 alkylene-O-C1-C30 alkyl group, substituted or unsubstituted C3-C30 heterocycloalkyl group, substituted or unsubstituted C6-C60 aryl group, substituted or unsubstituted C1-C60 heteroaryl group, substituted or unsubstituted C7-C60 aralkyl group, substituted or unsubstituted C6-C60 aryloxy group, and substituted or unsubstituted C6-C60 arylthio group, provided that at least one of R₁ and R₂ is not hydrogen atom.

More concretely, in the above formula 1, R₁ and R₂ may be each independently selected from the group consisting of hydrogen atom, halogen atom, substituted or unsubstituted C1-C12 alkyl group, substituted or unsubstituted C2-C12 alkenyl group, substituted or unsubstituted C2-C12 alkynyl group, substituted or unsubstituted C1-C10 alkoxy group, substituted or unsubstituted C3-C12 cycloalkyl group, substituted or unsubstituted C1-C12 alkylene-O-C1-C12 alkyl group, substituted or unsubstituted C3-C12 heterocycloalkyl group, substituted or unsubstituted C6-C20 aryl group, substituted or unsubstituted C1-C20 heteroaryl group, substituted or unsubstituted C7-C20 aralkyl group, substituted or unsubstituted C6-C20 aryloxy group, and substituted or unsubstituted C6-C20 arylthio group, provided that at least one of R₁ and R₂ is not hydrogen atom.

Even more concretely, in the above formula 1, R₁ and R₂ may be each independently selected from the group consisting of hydrogen atom, halogen atom, substituted or unsubstituted C1-C6 alkyl group, substituted or unsubstituted C2-C6 alkenyl group, substituted or unsubstituted C2-C6 alkynyl group, substituted or unsubstituted C1-C6 alkoxy group, substituted or unsubstituted C3-C6 cycloalkyl group, substituted or unsubstituted C1-C6 alkylene-O-C1-C6 alkyl group, substituted or unsubstituted C3-C6 heterocycloalkyl group, substituted or unsubstituted C6-C10 aryl group, substituted or unsubstituted C1-C10 heteroaryl group, substituted or unsubstituted C7-C10 aralkyl group, substituted or unsubstituted C6-C10 aryloxy group, and substituted or unsubstituted C6-C10 arylthio group, provided that at least one of R₁ and R₂ is not hydrogen atom.

As used herein, the expression of "substituted or unsubstituted" for any group means that the group is unsubstituted, or substituted with one or more substituents selected from halogen atom, C1-C6 alkyl group or halogenated C1-C6 alkyl group.

In an embodiment, the compound represented by the above formula 1 may be selected from the group consisting of compounds represented by the following formulas 2 to 4: 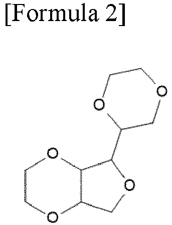 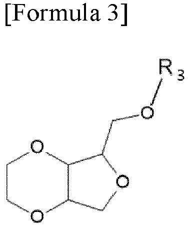 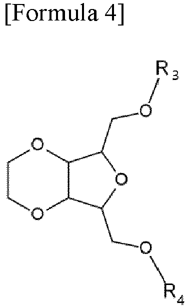 wherein R₃ and R₄ are each independently substituted or unsubstituted C1-C30 alkyl group, more concretely substituted or unsubstituted C1-C12 alkyl group, and even more concretely substituted or unsubstituted C1-C6 alkyl group.

More concretely, the compound represented by the above formula 2 may be selected from the group consisting of compounds represented by the following formulas 2-1 to 2-3; the compound represented by the above formula 3 may be a compound represented by the following formula 3-1 or 3-2; and the compound represented by the above formula 4 may be a compound represented by the following formula 4-1 or 4-2:

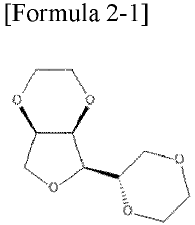

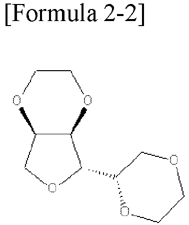

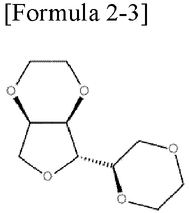

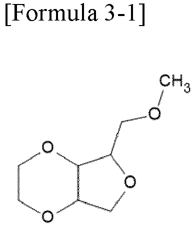

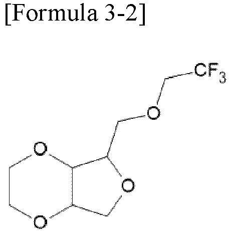

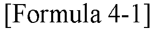

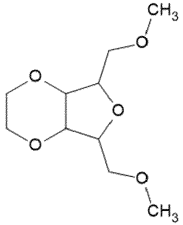

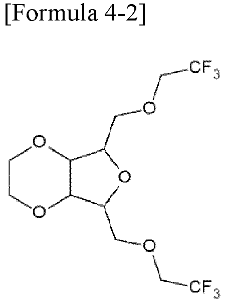

The other aspect of the present invention provides a method for preparing a compound represented by the above formula 2, the method comprising a step of heating dianhydrosugar hexitol-ethylene glycol, which is a compound in which ethylene glycol is added to the hydroxy groups at both ends of dianhydrosugar hexitol, in the presence of an acid catalyst.

Anhydrosugar alcohol generally means any material obtained by removing one or more water molecules from a compound called hydrogenated sugar or sugar alcohol obtained by adding hydrogen to the reductive end group in sugar, and dianhydrosugar hexitol is an anhydrosugar alcohol formed by removing two molecules of water from inside of hexitol, and it has a diol form with two hydroxyl groups in the molecule and can be produced by using hexitol derived from starch.

In an embodiment, the dianhydrosugar hexitol may be 1,4:3,6-dianhydrohexitol, and more concretely, it may be isosorbide, isomannide, isoidide or a mixture of two or more of the foregoing.

In an embodiment, the dianhydrosugar hexitol-ethylene glycol may be that prepared by addition reaction of dianhydrosugar hexitol and ethylene oxide of the following structure.

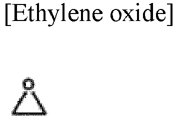

In an embodiment, the addition reaction of the dianhydrosugar hexitol and ethylene oxide may be conducted, for example, at a temperature of 100°C or higher, more concretely at a temperature of 100°C to 140°C for 1 hour or longer, more concretely 1 hour to 5 hours, but it is not limited thereto.

In an embodiment, the addition reaction of the dianhydrosugar hexitol and ethylene oxide can be conducted with using 2 moles or more of ethylene oxide, based on 1 mole of dianhydrosugar hexitol.

More concretely, in the addition reaction of the dianhydrosugar hexitol and ethylene oxide, the amount of ethylene oxide used per 1 mole of dianhydrosugar hexitol may be 2 moles or more, 3 moles or more, 4 moles or more, or 5 moles or more, and it also may be 25 moles or less, 20 moles or less, 15 moles or less, or 10 moles or less. If the amount of ethylene oxide used per 1 mole of dianhydrosugar hexitol in the addition reaction of the dianhydrosugar hexitol and ethylene oxide is less than 2 moles, the compound of the above formula 1 may not be prepared, and in contrast, if the amount is greater than 25 moles, it may result in a decrease in economic feasibility due to an increase in manufacturing costs

In an embodiment, the compound represented by the above formula 2-1 may be prepared by heating a product of the addition reaction using 2 moles or more of ethylene oxide per 1 mole of isosorbide in the presence of an acid catalyst.

In an embodiment, the compound represented by the above formula 2-2 may be prepared by heating a product of the addition reaction using 2 moles or more of ethylene oxide per 1 mole of isomannide in the presence of an acid catalyst.

In an embodiment, the compound represented by the above formula 2-3 may be prepared by heating a product of the addition reaction using 2 moles or more of ethylene oxide per 1 mole of isoidide in the presence of an acid catalyst.

In an embodiment, the acid catalyst may be an inorganic acid or an organic acid catalyst, and more concretely, may be selected from sulfuric acid, hydrochloric acid, hydrobromic acid, phosphoric acid, methanesulfonic acid, benzenesulfonic acid, para-toluenesulfonic acid, or a combination thereof, but it is limited thereto.

In an embodiment, the amount of acid catalyst used in the addition reaction of the dianhydrosugar hexitol and ethylene oxide based on 100 parts by weight of dianhydrosugar hexitol may be, for example, 0.01 part by weight or more, 0.02 part by weight or more, or 0.05 part by weight or more, and it also may be 5 parts by weight or less, 2 parts by weight or less, or 1 part by weight or less, but it is limited thereto.

Another aspect of the present invention provides a method for preparing a compound represented by the above formula 3 or 4, the method comprising a step of heating a compound represented by the following formula A or B, respectively, and an alkylating agent in the presence of a base catalyst:

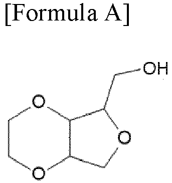

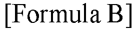

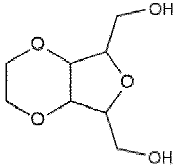

In an embodiment, the alkylating agent may be selected from alkyl halide of formula R-X [wherein R is a substituted or unsubstituted C1-C30 alkyl group (more concretely, a substituted or unsubstituted C1-C12 alkyl group, and even more concretely, a substituted or unsubstituted C1-C6 alkyl group), and X is a halogen atom (e.g., F, Cl, Br or I)], aromatic sulfonate having substituted or unsubstituted C1-C30 alkyl group, or combination thereof, but it is limited thereto.

More concretely, the alkyl halide may be methyl iodide, and the aromatic sulfonate having substituted or unsubstituted C1-C30 alkyl group may be toluenesulfonate having C1-C30 alkyl group substituted with halogen atom (e.g., F), but it is limited thereto.

In an embodiment, the base catalyst may be selected from inorganic base catalyst, organic base catalyst, or combination thereof, and more concretely, may be selected from sodium hydride, hydrogen iodide, hydrogen sulfide, aluminum hydride, or combination thereof, but it is limited thereto.

In an embodiment, the amount of base catalyst used in the reaction of the compound represented by the above formula A or B and alkylating agent based on 100 parts by weight of the compound of formula A or B may be, for example, 1 part by weight or more, 2 parts by weight or more, or 5 parts by weight or more, and it also may be 80 parts by weight or less, 60 parts by weight or less, or 50 parts by weight or less, but it is limited thereto.

In an embodiment, the reaction of the compound represented by the above formula A or B and alkylating agent may be conducted, for example, at a temperature of 40°C or higher, more concretely at a temperature of 40°C to 80°C for 5 hours or longer, more concretely 5 hours to 15 hours, but it is not limited thereto.

In an embodiment according to the present invention, the compound represented by the above formula 1 may be effectively used as an electrolyte additive in a non-aqueous electrolyte composition for secondary battery, particularly, non-aqueous electrolyte composition for lithium-sulfur secondary battery, as explained below as another aspect of the present invention. However, use of the compound of the present invention is never be limited thereto, and the compound represented by the above formula 1 may be used for various applications (for example, surfactant, penetration enhancer of active ingredients in cosmetics, antibacterial agent, etc.) other than such electrolyte additive for secondary battery.

### [Electrolyte additive and non-aqueous electrolyte composition]

Still another aspect of the present invention relates to an electrolyte additive comprising the compound represented by the above formula 1.

In an embodiment, the electrolyte additive may consist of the compound represented by the above formula 1 alone.

In an embodiment, the electrolyte additive may further comprise one or more components other than the compound represented by the above formula 1. For such a further component, a component that can be used as an additive for secondary battery may be used without special limitation.

More concretely, as the component other than the compound represented by the above formula 1, the electrolyte additive may further comprise nitric (or nitrous) acid-based compound. Such a nitric (or nitrous) acid-based compound can form a stable film on the anode (lithium-containing electrode) of lithium-sulfur secondary battery and improve the charge/discharge efficiency.

In an embodiment, for example, the nitric (or nitrous) acid-based compound may be selected from the group consisting of inorganic nitric acid compound (e.g., lithium nitrate (LiNOs), lithium nitrite (LiNO₂), etc.), organic nitric acid compound (e.g., nitromethane (CH₃NO₂), methyl nitrate (CH₃NO₃), etc.), or mixture thereof, but it is not limited thereto.

In an embodiment, when the electrolyte additive comprises the compound represented by the above formula 1 and the other component (for example, the nitric (or nitrous) acid-based compound), the amount of the other component based on 1 part by weight of the compound represented by the above formula 1 contained in the electrolyte additive may be 0.1 to 2 parts by weight, and more concretely, it may be 0.1 part by weight or more, 0.2 part by weight or more, or 0.3 part by weight or more, and also may be 2 parts by weight or less, 1.8 parts by weight or less, or 1.5 parts by weight or less, but it is not limited thereto.

Still another aspect of the present invention relates to a non-aqueous electrolyte composition comprising: a non-aqueous electrolyte solvent; lithium salt; and the electrolyte additive of the present invention, wherein the amount of the electrolyte additive is from 0.1 to 10 parts by weight, based on total 100 parts by weight of the electrolyte composition.

If the amount of the electrolyte additive in total 100 parts by weight of the non-aqueous electrolyte composition of the present invention is less than 0.1 part by weight, when using such an electrolyte composition, the effect of improving the life retention rate (or capacity retention rate) of the lithium-sulfur secondary battery may be insufficient, and in contrast, if the amount of the electrolyte additive is greater than 10 parts by weight, when using such an electrolyte composition, the film may be thickened, and as a result, resistance may increase, resulting in decrease of the life retention rate (or capacity retention rate) of the lithium-sulfur secondary battery.

More concretely, the amount of the electrolyte additive in total 100 parts by weight of the non-aqueous electrolyte composition of the present invention may be 0.1 part by weight or more, 0.5 part by weight or more, 1 part by weight or more, or 1.2 parts by weight or more, and it also may be 10 parts by weight or less, 9.5 parts by weight or less, 9 parts by weight or less, or 8.9 parts by weight or less.

The non-aqueous electrolyte solvent contained in the non-aqueous electrolyte composition of the present invention serves as a medium through which ions involved in the electrochemical reaction of lithium-sulfur secondary battery can move.

In an embodiment, the non-aqueous electrolyte solvent may be linear ether, cyclic ether, or combination thereof.

In an embodiment, for example, the non-aqueous electrolyte solvent may be selected from the group consisting of dimethyl ether, diethyl ether, dipropyl ether, dibutyl ether, diisobutyl ether, ethylmethyl ether, ethylpropyl ether, ethyl tert-butyl ether, dimethoxymethane, trimethoxymethane, dimethoxyethane, diethoxyethane, dimethoxypropane, diethylene glycol dimethyl ether, diethylene glycol diethyl ether, triethylene glycol dimethyl ether, tetraethylene glycol dimethyl ether, ethylene glycol divinyl ether, diethylene glycol divinyl ether, triethylene glycol divinyl ether, dipropylene glycol dimethylene ether, butylene glycol ether, diethylene glycol ethyl methyl ether, diethylene glycol isopropyl methyl ether, diethylene glycol butyl methyl ether, diethylene glycol tert-butyl ethyl ether, ethylene glycol ethyl methyl ether, dioxolane, methyldioxolane, dimethyldioxolane, vinyldioxolane, methoxydioxolane, ethylmethyldioxolane, oxane, dioxane, trioxane, tetrahydrofuran, methyltetrahydrofuran, dimethyltetrahydrofuran, dimethoxytetrahydro Furan, ethoxytetrahydrofuran, dihydropyran, tetrahydropyran, furan, methylfuran, isosorbide dimethyl ether, or mixture thereof, but it is not limited thereto.

In an embodiment, the lithium salt contained in the non-aqueous electrolyte composition of the present invention may be selected from the group consisting of LiSCN, LiBr, LiI, LiPF₆, LiBF₄, LiSbF₆, LiAsF₆, LiCH₃SO₃, LiCF₃SO₃, LiClO₄, LiBPh₄, LiC(CF₃SO₂)₃, LiN(CF₃SO₂)₂, LiN(C₂F₅SO₂)₂, LiN(SFO₂)₂, LiN(CF₃CF₂SO₂)₂, Li₂S, LiSn (wherein n=an integer of 1 to 8), or mixture thereof, but it is not limited thereto. In the above, Ph means phenyl.

In an embodiment, the molar concentration of the lithium salt in the non-aqueous electrolyte composition of the present invention may be 0.5 M to 5 M. If the molar concentration of the lithium salt in the electrolyte composition is less than 0.5 M, lowering of capacity retention rate may occur, and in contrast, if it is greater than 5 M, it is not preferable because the lithium salt may be eluted and economic feasibility decreases.

More concretely, the molar concentration of the lithium salt in the non-aqueous electrolyte composition of the present invention may be 0.5 M or more, 0.6 M or more, 0.7 M or more, 0.8 M or more, 0.9 M or more, or 1 M or more, and it also may be 5 M or less, 4.8 M or less, 4.6 M or less, 4.4 M or less, or 4.2 M or less.

### [Secondary battery]

Still another aspect of the present invention relates to a secondary battery (in particular, a lithium-sulfur secondary battery) comprising: a cathode; an anode; a separation membrane; and the non-aqueous electrolyte composition of the present invention.

In an embodiment, the secondary battery of the present invention comprises a cathode and an anode placed opposite each other; a separation membrane between the cathode and anode; and the non-aqueous electrolyte composition of the present invention which is impregnated into the cathode, anode and separation membrane, and has ion conductivity.

The cathode comprises a cathode current collector; and a cathode active material layer formed on the cathode current collector.

The cathode current collector may be any one that can be used as a current collector in this field of art, and concretely, it is preferable to use foamed aluminum, foamed nickel, etc., having good conductivity.

The cathode active material layer may comprise, as a cathode active material, elemental sulfur (Ss), sulfur-based compound or mixture thereof. Concretely, the sulfur-based compound may be Li₂Sₙ (n=1), organic sulfur compound, or carbon-sulfur polymer ((C₂Sₓ)ₙ: x=2.5 to 50, n=2), etc.

Since such a sulfur material alone does not have electric conductivity, the cathode active material layer comprises a conductive material.

The conductive material may be porous. Thus, as the conductive material, any having porosity and conductivity may be used without limitation, and for example, a carbon-based material with porosity may be used. For such a carbon-based material, carbon black, graphite, graphene, activated carbon, carbon fiber, etc. may be used. In addition, metallic fiber such as metal mesh; metallic powder such as copper, silver, nickel, aluminum, etc.; or organic conductive material such as polyphenylene derivative may also be used. The above conductive materials may be used alone or in combination.

In an embodiment, the cathode active material layer may further comprise a binder in order to improve the bonding between the cathode active material and the conductive material and the bonding between the cathode material layer and the cathode current collector.

The binder may comprise thermoplastic resin or thermosetting resin. For example, it is possible to use polyethylene, polyethylene oxide, polypropylene, polytetrafluoroethylene (PTFE), polyvinylidene fluoride (PVDF), styrene-butadiene rubber, tetrafluoroethylene-perfluoro alkyl vinyl ether copolymer, vinylidene fluoride-hexafluoropropylene copolymer, vinylidene fluoride-chlorotrifluoroethylene copolymer, ethylene-tetrafluoroethylene copolymer, polychlorotrifluoroethylene, vinylidene fluoride-pentafluoropropylene copolymer, propylene-tetrafluoroethylene copolymer, ethylene-chlorotrifluoroethylene copolymer, vinylidene fluoride-hexafluoropropylene-tetrafluoroethylene copolymer, vinylidene fluoride-perfluoromethylvinyl ether-tetrafluoroethylene copolymer, ethylene-acrylic acid copolymer, etc. alone or in combination, but it is not limited thereto, and it is possible to use any one that can be used as a binder in this field of art.

A cathode as above can be manufactured according to a conventional method, and concretely, it can be manufactured by applying a composition for forming a cathode active material layer prepared by mixing a cathode active material, a conductive material and a binder in an organic solvent, on a cathode current collector and drying it; and optionally performing compression molding on the cathode current collector to improve electrode density. At this time, it is preferable to use an organic solvent which can disperse the cathode active material, binder and conductive material uniformly and is evaporated easily. Concretely, it is possible to use an organic solvent selected from acetonitrile, methanol, ethanol, tetrahydrofuran, water, isopropyl alcohol, or combination thereof.

The anode may comprise an anode current collector; and an anode active material layer formed on the anode current collector, or it may be a lithium metal plate alone.

The anode current collector is for supporting the anode active material, and there is no special limitation thereto as long as it has good conductivity and being electrochemically stable in the voltage range of lithium metal battery, and for example, copper, stainless steel, aluminum, nickel, titanium, palladium, calcined carbon, surface-treated copper or stainless steel with carbon, nickel, silver, etc., aluminum-cadmium alloy, etc. may be used.

The anode current collector may form fine irregularities on its surface to strengthen the bonding force with the anode active material layer, and it may be used in various forms such as film, sheet, foil, mesh, net, porous material, foamed material or non-woven material, etc.

The anode active material layer may comprise, as an anode active material, a material capable of reversibly intercalating or deintercalating lithium ion (Li⁺); a material capable of reacting with lithium ion to reversibly form a lithium-containing compound; lithium metal; or lithium alloy.

The material capable of reversibly intercalating or deintercalating lithium ion (Li⁺) may be, for example, crystalline carbon, amorphous carbon, or mixture thereof. The material capable of reacting with lithium ion to reversibly form a lithium-containing compound may be, for example, tin oxide, titanium nitrate or silicon. The lithium alloy may be, for example, alloy of lithium (Li) with metal selected from the group consisting of sodium (Na), potassium (K), rubidium (Rb), cesium (Cs), francium (Fr), beryllium (Be), magnesium (Mg), calcium (Ca), strontium (Sr), barium (Ba), radium (Ra), aluminum (Al) and tin (Sn).

In a preferable embodiment, the anode active material may be lithium metal, and concretely, it may be in a form of lithium metal thin film or lithium metal powder.

There is no special limitation to the method of forming the anode active material layer, and any method of forming a layer or film conventionally used in this field of art can be used. For example, a method such as compression, coating, deposition, etc. can be used. In addition, the anode in the present invention also includes a case where a battery is assembled without a lithium thin film on the anode current collector and a lithium metal thin film is formed on metal plate through initial charging.

The non-aqueous electrolyte composition of the present invention is the same as explained above, and it comprises lithium ion and is for causing an electrochemical oxidation or reduction reaction at the cathode and anode through lithium ions.

Injection of the non-aqueous electrolyte composition may be performed at an appropriate stage during the manufacturing process of the electrochemical device, depending on the manufacturing process and required properties of the final product. That is, it can be applied before assembling electrochemical device or at the final stage in assembling electrochemical device.

Between the cathode and anode explained above, a separation membrane may be comprised additionally. The separation membrane is for physically separating the two electrodes in the secondary battery (particularly, lithium-sulfur secondary battery) of the present invention, and any one conventionally used as a separation membrane in secondary battery (particularly, lithium-sulfur secondary battery) can be used without special limitation, and it is particularly preferable to use a separation membrane having low resistance to ion movement in the electrolyte composition and good impregnation (humidification) ability for the electrolyte composition.

The separation membrane may consist of porous material, and any porous material conventionally used for electrochemical device can be used, and for example, porous membrane or non-woven fabric may be used, but it is not especially limited thereto.

A concrete example of the porous membrane may be polyolefin-based porous membrane. The polyolefin-based porous membrane may be a membrane formed by using polyolefin-based polymer such as polyethylene (e.g., high density polyethylene, linear low density polyethylene, low density polyethylene, ultra-high molecular weight polyethylene, etc.), polypropylene, polybutylene, polypentene, etc. alone, or a membrane formed from a polymer mixture thereof.

The non-woven fabric may be, in addition to polyolefin-based non-woven fabric, for example, a non-woven fabric formed by using polyethylene terephthalate, polybutylene terephthalate, polyester, polyacetal, polyamide, polycarbonate, polyimide, poly(ether ether ketone), poly(ether sulfone), poly(phenylene oxide), poly(phenylene sulfide), polyethylene naphthalate, etc. alone, or a non-woven fabric formed from a polymer mixture thereof. The structure of the non-woven fabric may be sponbond non-woven fabric consisting of long fiber or melt blown non-woven fabric.

There is no special limitation to the thickness of the porous material, but it may be 1 to 100 µm, preferably 5 to 50 µm.

There is no special limitation to the size and porosity of pores present in the porous material, but they may be 0.001 to 50 µm and 10 to 95%, respectively.

The secondary battery (particularly, lithium-sulfur secondary battery) according to the present invention may be manufactured through a process of lamination and stack of the separation membrane and electrodes, and folding them, in addition to a general winding process.

There is no special limitation to the shape of the secondary battery, and it may be manufactured in various forms such as cylindrical type, stacked type, coin-shaped type, etc.

The present invention is explained in more detail through the following Examples and Comparative Examples. However, the scope of the present invention is not limited thereby in any manner.

### EXAMPLES

### <Preparation of dianhydrosugar hexitol-ethylene glycol>

In the preparation examples of dianhydrosugar hexitol-ethylene glycol below, the addition reaction scheme of dianhydrosugar hexitol (isosorbide) and ethylene oxide according to stoichiometry is as follows: 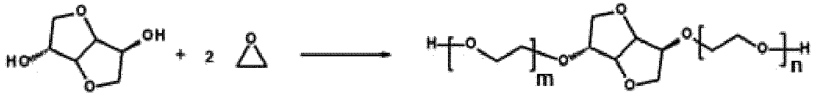 (wherein m = 1, n = 1)

### Preparation Example A1: Addition reaction performed using 5 moles of ethylene oxide based on 1 mole of isosorbide

146g of isosorbide and 0.15g of phosphoric acid (85%) as an acid component were put into a reactor that could be pressurized, and the inside of the reactor was substituted with nitrogen and heated up to 100°C and the moisture in the reactor was removed by pressure reduction under vacuum. Then, while firstly adding 88g of ethylene oxide slowly thereto, the reaction was conducted at 100 to 140°C for 2 to 3 hours. At that time, the reaction temperature was controlled so as not to exceed 140°C. Thereafter, the inside of the reactor was cooled to 50°C, and then 0.3g of potassium hydroxide was added to the reactor, the inside of the reactor was substituted with nitrogen and heated up to 100°C and the moisture in the reactor was removed by pressure reduction under vacuum. Then, while secondly adding 132g of ethylene oxide slowly thereto, the reaction was conducted at 100 to 140°C for 2 to 3 hours. After completing the reaction, the inside of the reactor was cooled to 50°C, 4.0g of Ambosol MP20 as adsorbent was added thereto, and the inside of the reactor was reheated and agitated at 100°C to 120°C for 1 to 5 hours to remove residual metal ions. At that time, the inside of the reactor was substituted with nitrogen and/or pressure reduction under vacuum was carried out. After confirming that no metal ions were detected, the inside of the reactor was cooled to 60°C to 90°C and the remaining byproduct was removed to obtain 362g of the addition reaction product in transparent liquid form.

### Preparation Example A2: Addition reaction performed using 10 moles of ethylene oxide based on 1 mole of isosorbide

Excepting that the secondly added amount of ethylene oxide was changed from 132g to 352g, the same method as Preparation Example A1 was conducted to obtain 551g of the addition reaction product in transparent liquid form.

### Preparation Example A3: Addition reaction performed using 2 moles of ethylene oxide based on 1 mole of isosorbide

Excepting that the firstly added amount of ethylene oxide was changed from 88g to 44g and the secondly added amount of ethylene oxide was changed from 132g to 44g, the same method as Preparation Example A1 was conducted to obtain 227g of the addition reaction product in transparent liquid form.

### <Preparation of compound of formula 2>

In the preparation examples of compound below, the reaction scheme of forming the target compound ((4aR,5R,7aR)-5-[(2S)-1,4-dioxan-2-yl]-2,3,4a,5,7,7a-hexahydrofuro[3,4-b][1,4]dioxine) from dianhydrosugar hexitol-ethylene glycol is as follows: 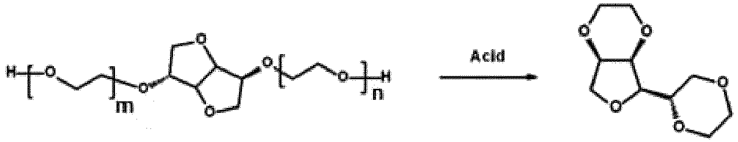 (wherein m = 1, n = 1)

### Example A1: Preparation of the target compound using the addition reaction product of Preparation Example A1

In a three-neck glass reactor equipped with a vacuum pump and micro sublimation device sequentially connected in series, 200 g of the addition reaction product obtained in Preparation Example A1 and 5 g of sulfuric acid were added, and the reaction proceeded while heating to 130°C. After heating and stirring for 1 hour, the pressure was slowly reduced to 50 mbar using a vacuum pump to remove decomposed gas and 1,4-dioxane, and 35 g of the target compound in needle-shaped crystal was obtained by using the micro sublimation device. The analysis results of the obtained target compound are as follows:
¹H NMR (400 MHz, CDCl₃): d 4.39 (td, J = 8.6, 4.0 Hz, 1H), 4.18 (t, J = 8.6 Hz, 1H), 3.96 (m, 1H), 3.96-3.83 (m, 5H), 3.81-3.74 (m, 3H), 3.71 (m, 1H), 3.66-3.58 (m, 2H), 3.50 (m, 1H), 3.33 (m, 1H).
¹³C NMR (100 MHz, CDCl₃): d 80.1, 74.7, 73.9, 72.4, 67.3, 66.6, 66.3, 64.6, 64.4, 59.6. Mass (ESI): 217.10 (M+1).

### Example A2: Preparation of the target compound using the addition reaction product of Preparation Example A2

Excepting that 200g of the addition reaction product obtained in Preparation Example A2 was used instead of the addition reaction product obtained in Preparation Example A1, the same method as Example A1 was conducted to obtain 19g of the target compound in needle-shaped crystal. The analysis results of the obtained target compound were the same as those in Example A1.

### Example A3: Preparation of the target compound using the addition reaction product of Preparation Example A3

Excepting that 200g of the addition reaction product obtained in Preparation Example A3 was used instead of the addition reaction product obtained in Preparation Example A1, the same method as Example A1 was conducted to obtain 12g of the target compound in needle-shaped crystal. The analysis results of the obtained target compound were the same as those in Example A1.

### <Preparation of compound of formula 3-1>

### Example A4: Preparation of the target compound using monohydroxy furodioxine

In the preparation example of compound below, the reaction scheme of forming the target compound (5-(methoxymethyl)hexahydrofuro[3,4-b][1,4]dioxine) from monohydroxy furodioxine is as follows:

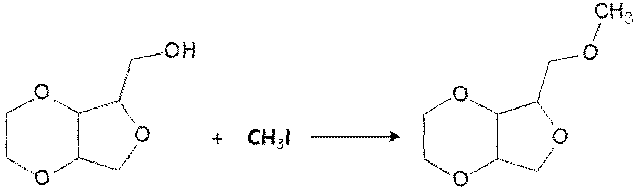

In a 1,000 mL flask, 400 mL of tetrahydrofuran was added, and 80 g of the starting material ([hexahydrofuro[3,4-b][1,4]dioxin-5-yl]methanol) and 36 g of sodium hydride were added thereto, and then the temperature inside of the flask was maintained at 0°C for 10 minutes. After stirring at 0°C for 30 minutes, 90 g of methyl iodide was added and the temperature was elevated to 60°C, and then the reaction proceeded while stirring for 12 hours. After completing the reaction, the resulting mixture was filtered and dried under vacuum to obtain 56g of the target compound of the above formula 3-1 (5-(methoxymethyl)hexahydrofuro[3,4-b][1,4]dioxine).

¹H NMR (400 MHz, DMSO-d₆): d 4.80 (td, 1H), 4.51 (t, 1H), 4.00-3.75 (m, 2H), 3.76-3.66 (m, 4H), 3.75 (m, 1H), 3.60-3.35 (m, 2H), 3.23 (s, 3H).

¹³C NMR (100 MHz, DMSO-d₆): d 91.1, 89.6, 86.1, 72.0, 62.7, 61.4, 61.1, 59.6. Mass (ESI): 174.2 (M+1).

### <Preparation of compound of formula 3-2>

### Example A5: Preparation of the target compound using monohydroxy furodioxine

Excepting that 120g of 2,2,2-trifluoroethyl p-toluenesulfonate was used instead of 90 g of methyl iodide, the same method as Example A4 was conducted to obtain 82g of the target compound of the above formula 3-2 (5-((2,2,2-trifluoroethoxy)methyl)hexahydrofuro[3,4-b][1,4]dioxine).

¹H NMR (400 MHz, DMSO-d₆): d 4.80 (td, 1H), 4.51 (td, 1H), 4.00-3.75 (m, 2H), 3.76-3.66 (m, 4H), 3.75 (m, 1H), 3.60-3.35 (m, 2H), 3.23 (s, 2H).

¹³CNMR(100MHz,DMSO-d₆): d 128.5, 123.5, 91.1, 89.6, 86.1, 62.7, 61.4, 61.1, 51.6. Mass (ESI): 242.2 (M+1).

### <Preparation of compound of formula 4-1>

### Example A6: Preparation of the target compound using dihydroxy furodioxine

Excepting that 90g of 2,5-anhydro-3,4-O-(1,2-ethanediyl)mannitol was used as the starting material instead of 80 g of [hexahydrofuro[3,4-b][1,4]dioxin-5-yl]methanol, the same method as Example A4 was conducted to obtain 66g of the target compound of the above formula 4-1 (5,7-bis(methoxymethyl)hexahydrofuro[3,4-b][1,4]dioxine).

¹H NMR (400 MHz, DMSO-d₆): d 4.80 (td, 2H), 3.75 (m, 2H), 3.76-3.66 (m, 4H), 3.60-3.35 (m, 4H), 3.23 (s, 6H).

¹³C NMR (100 MHz, DMSO-d₆): d 88.9, 82.1, 72.3, 61.4, 59.6.

Mass (ESI): 218.26 (M+1).

### <Preparation of compound of formula 4-2>

### Example A7: Preparation of the target compound using dihydroxy furodioxine

Excepting that 90g of 2,5-anhydro-3,4-O-(1,2-ethanediyl)mannitol was used as the starting material instead of 80 g of [hexahydrofuro[3,4-b][1,4]dioxin-5-yl]methanol and 120g of 2,2,2-trifluoroethyl p-toluenesulfonate was used instead of 90 g of methyl iodide, the same method as Example A4 was conducted to obtain 83g of the target compound of the above formula 4-2 (5,7-bis((2,2,2-trifluoroethoxy)methyl)hexahydrofuro[3,4-b][1,4]dioxine).

¹H NMR (400 MHz, DMSO-d₆): d 4.92 (dd, 4H), 4.80 (td, 2H), 3.75 (m, 2H), 3.76-3.66 (m, 4H), 3.60-3.35 (m, 4H), 3.23 (s, 6H).

¹³C NMR (100 MHz, DMSO-d₆): d 128.5, 121.6, 88.9, 82.1, 72.3, 61.4, 51.9.

Mass (ESI): 354.25 (M+1).

### <Preparation of non-aqueous electrolyte composition>

### Example B1: Preparation of electrolyte composition containing, as additive, 1 part by weight of LiNO₃ and 2 parts by weight of the target compound obtained in Example A1

In an electrolyte liquid comprising a non-aqueous electrolyte solvent consisting of 1,2-dimethoxyethane and 1,3-dioxolane (volume ratio = 50:50) and 1.0 M of LiN(CF₃SO₂)₂, based on total 100 parts by weight of the electrolyte composition, 1 part by weight of LiNO₃ and 2 parts by weight of the target compound obtained in Example A1 were mixed as additive to prepare 10 mL of the electrolyte composition.

### Example B2: Preparation of electrolyte composition containing, as additive, 4.5 parts by weight of LiNO₃ and 4.4 parts by weight of the target compound obtained in Example A2

In an electrolyte liquid comprising a non-aqueous electrolyte solvent consisting of 1,2-dimethoxyethane and 1,3-dioxolane (volume ratio = 50:50) and 4.2 M of LiN(CF₃SO₂)₂, based on total 100 parts by weight of the electrolyte composition, 4.5 parts by weight of LiNO₃ and 4.4 parts by weight of the target compound obtained in Example A2 were mixed as additive to prepare 10 mL of the electrolyte composition.

### Example B3: Preparation of electrolyte composition containing, as additive, 1 part by weight of CH₃NO₃ and 4.3 parts by weight of the target compound obtained in Example A3

In an electrolyte liquid comprising a non-aqueous electrolyte solvent consisting of 1,2-dimethoxyethane and 1,3-dioxolane (volume ratio = 50:50) and 1.0 M of LiN(CF₃SO₂)₂, based on total 100 parts by weight of the electrolyte composition, 1 part by weight of CH₃NO₃ and 4.3 parts by weight of the target compound obtained in Example A3 were mixed as additive to prepare 10 mL of the electrolyte composition.

### Example B4: Preparation of electrolyte composition containing, as additive, 0.5 part by weight of LiNO₂ and 0.7 part by weight of the target compound obtained in Example A1

In an electrolyte liquid comprising a non-aqueous electrolyte solvent consisting of 1,2-dimethoxyethane and 1,3-dioxolane (volume ratio = 50:50) and 0.4 M of LiPF₆, based on total 100 parts by weight of the electrolyte composition, 0.5 part by weight of LiNO₂ and 0.7 part by weight of the target compound obtained in Example A1 were mixed as additive to prepare 10 mL of the electrolyte composition.

### Example B5: Preparation of electrolyte composition containing, as additive, 1 part by weight of LiNO₃ and 2 parts by weight of the target compound obtained in Example A1

In an electrolyte liquid comprising a non-aqueous electrolyte solvent consisting of 1,2-dimethoxyethane alone and 1.0 M of LiN(CF₃SO₂)₂, based on total 100 parts by weight of the electrolyte composition, 1 part by weight of LiNO₃ and 2 parts by weight of the target compound obtained in Example A1 were mixed as additive to prepare 10 mL of the electrolyte composition.

### Example B6: Preparation of electrolyte composition containing, as additive, 1 part by weight of LiNO₃ and 2 parts by weight of the target compound obtained in Example A1

In an electrolyte liquid comprising a non-aqueous electrolyte solvent consisting of tetraethylene glycol dimethyl ether and 1,3-dioxolane (volume ratio = 25:75) and 1.0 M of LiN(CF₃SO₂)₂, based on total 100 parts by weight of the electrolyte composition, 1 part by weight of LiNO₃ and 2 parts by weight of the target compound obtained in Example A1 were mixed as additive to prepare 10 mL of the electrolyte composition.

### Example B7: Preparation of electrolyte composition containing, as additive, 1 part by weight of LiNO₃ and 2 parts by weight of the target compound obtained in Example A4

In an electrolyte liquid comprising a non-aqueous electrolyte solvent consisting of 1,2-dimethoxyethane and 1,3-dioxolane (volume ratio = 50:50) and 1.0 M of LiN(CF₃SO₂)₂, based on total 100 parts by weight of the electrolyte composition, 1 part by weight of LiNO₃ and 2 parts by weight of the target compound obtained in Example A4 were mixed as additive to prepare 10 mL of the electrolyte composition.

### Example B8: Preparation of electrolyte composition containing, as additive, 1 part by weight of LiNO₃ and 2 parts by weight of the target compound obtained in Example A5

In an electrolyte liquid comprising a non-aqueous electrolyte solvent consisting of 1,2-dimethoxyethane and 1,3-dioxolane (volume ratio = 50:50) and 1.0 M of LiN(CF₃SO₂)₂, based on total 100 parts by weight of the electrolyte composition, 1 part by weight of LiNO₃ and 2 parts by weight of the target compound obtained in Example A5 were mixed as additive to prepare 10 mL of the electrolyte composition.

### Example B9: Preparation of electrolyte composition containing, as additive, 1 part by weight of LiNO₃ and 2 parts by weight of the target compound obtained in Example A6

In an electrolyte liquid comprising a non-aqueous electrolyte solvent consisting of 1,2-dimethoxyethane and 1,3-dioxolane (volume ratio = 50:50) and 1.0 M of LiN(CF₃SO₂)₂, based on total 100 parts by weight of the electrolyte composition, 1 part by weight of LiNO₃ and 2 parts by weight of the target compound obtained in Example A6 were mixed as additive to prepare 10 mL of the electrolyte composition.

### Example B10: Preparation of electrolyte composition containing, as additive, 1 part by weight of LiNO₃ and 2 parts by weight of the target compound obtained in Example A7

In an electrolyte liquid comprising a non-aqueous electrolyte solvent consisting of 1,2-dimethoxyethane and 1,3-dioxolane (volume ratio = 50:50) and 1.0 M of LiN(CF₃SO₂)₂, based on total 100 parts by weight of the electrolyte composition, 1 part by weight of LiNO₃ and 2 parts by weight of the target compound obtained in Example A7 were mixed as additive to prepare 10 mL of the electrolyte composition.

### Comparative Example B1: Preparation of electrolyte composition containing, as additive, only 1 part by weight of LiNO₃

In an electrolyte liquid comprising a non-aqueous electrolyte solvent consisting of 1,2-dimethoxyethane and 1,3-dioxolane (volume ratio = 50:50) and 1.0 M of LiN(CF₃SO₂)₂, based on total 100 parts by weight of the electrolyte composition, only 1 part by weight of LiNO₃ was mixed as additive to prepare 10 mL of the electrolyte composition.

### Comparative Example B2: Preparation of electrolyte composition containing 0.09 part by weight of additive (0.04 part by weight of LiNO₃ + 0.05 part by weight of the target compound obtained in Example A1)

In an electrolyte liquid comprising a non-aqueous electrolyte solvent consisting of 1,2-dimethoxyethane and 1,3-dioxolane (volume ratio = 50:50) and 1.0 M of LiN(CF₃SO₂)₂, based on total 100 parts by weight of the electrolyte composition, 0.04 part by weight of LiNO₃ and 0.05 part by weight of the target compound obtained in Example A1 were mixed as additive to prepare 10 mL of the electrolyte composition.

### Comparative Example B3: Preparation of electrolyte composition containing 10.1 parts by weight of additive (5.5 parts by weight of LiNO₃ + 4.6 parts by weight of the target compound obtained in Example A1)

In an electrolyte liquid comprising a non-aqueous electrolyte solvent consisting of 1,2-dimethoxyethane and 1,3-dioxolane (volume ratio = 50:50) and 1.0 M of LiN(CF₃SO₂)₂, based on total 100 parts by weight of the electrolyte composition, 5.5 parts by weight of LiNO₃ and 4.6 parts by weight of the target compound obtained in Example A1 were mixed as additive to prepare 10 mL of the electrolyte composition.

### Comparative Example B4: Preparation of electrolyte composition containing 0.09 part by weight of additive (0.04 part by weight of LiNO₃ + 0.05 part by weight of the target compound obtained in Example A4)

In an electrolyte liquid comprising a non-aqueous electrolyte solvent consisting of 1,2-dimethoxyethane and 1,3-dioxolane (volume ratio = 50:50) and 1.0 M of LiN(CF₃SO₂)₂, based on total 100 parts by weight of the electrolyte composition, 0.04 part by weight of LiNO₃ and 0.05 part by weight of the target compound obtained in Example A4 were mixed as additive to prepare 10 mL of the electrolyte composition.

### Comparative Example B5: Preparation of electrolyte composition containing 10.1 parts by weight of additive (5.5 parts by weight of LiNO₃ + 4.6 parts by weight of the target compound obtained in Example A5)

In an electrolyte liquid comprising a non-aqueous electrolyte solvent consisting of 1,2-dimethoxyethane and 1,3-dioxolane (volume ratio = 50:50) and 1.0 M of LiN(CF₃SO₂)₂, based on total 100 parts by weight of the electrolyte composition, 5.5 parts by weight of LiNO₃ and 4.6 parts by weight of the target compound obtained in Example A5 were mixed as additive to prepare 10 mL of the electrolyte composition.

### Comparative Example B6: Preparation of electrolyte composition containing 0.09 part by weight of additive (0.04 part by weight of LiNO₃ + 0.05 part by weight of the target compound obtained in Example A6)

In an electrolyte liquid comprising a non-aqueous electrolyte solvent consisting of 1,2-dimethoxyethane and 1,3-dioxolane (volume ratio = 50:50) and 1.0 M of LiN(CF₃SO₂)₂, based on total 100 parts by weight of the electrolyte composition, 0.04 part by weight of LiNO₃ and 0.05 part by weight of the target compound obtained in Example A6 were mixed as additive to prepare 10 mL of the electrolyte composition.

### Comparative Example B7: Preparation of electrolyte composition containing 10.1 parts by weight of additive (5.5 parts by weight of LiNO₃ + 4.6 parts by weight of the target compound obtained in Example A7)

In an electrolyte liquid comprising a non-aqueous electrolyte solvent consisting of 1,2-dimethoxyethane and 1,3-dioxolane (volume ratio = 50:50) and 1.0 M of LiN(CF₃SO₂)₂, based on total 100 parts by weight of the electrolyte composition, 5.5 parts by weight of LiNO₃ and 4.6 parts by weight of the target compound obtained in Example A7 were mixed as additive to prepare 10 mL of the electrolyte composition.

### <Manufacture of lithium-sulfur secondary battery>

### Examples C1 to C10 and Comparative Examples C1 to C7

65 parts by weight of sulfur, 25 parts by weight of carbon black and 10 parts by weight of polyethylene oxide were mixed with acetonitrile to prepare a composition for forming cathode active material layer. At that time, the sum of amounts of sulfur, carbon black and polyethylene oxide in total was 100 parts by weight. The composition for forming cathode active material layer was coated on an aluminum current collector and dried to prepare a cathode. Also, lithium metal with a thickness of 450 µm was used as an anode. The prepared cathode and anode were positioned to face each other, and a polypropylene separation membrane was placed between them, and then each of the electrolyte compositions of Examples B1 to B10 and Comparative Examples B1 to B7 obtained above was charged therein to manufacture each of the lithium-sulfur secondary batteries of Examples C1 to C10 and Comparative Examples C1 to C7.

### <Analysis of charge/discharge characteristics of lithium-sulfur secondary battery>

The charge/discharge characteristics of the lithium-sulfur secondary batteries of Examples C1 to C10 and Comparative Examples C1 to C7 were measured by the following method, and the results are shown in Table 1 below.

### Evaluation of life retention at room temperature (Discharge Capacity)

Evaluation of life retention at room temperature was performed on the lithium-sulfur secondary batteries manufactured in Examples and Comparative Examples. Concretely, the manufactured lithium-sulfur secondary battery was discharged up to 1.8V by fixing at 0.36 mA in constant current mode at room temperature (25°C), and after 10 minutes from completion of discharge, it was charged at 0.36 mA in constant current mode until the operating voltage became 2.7V. One time of the above charge and one time of the above discharge were defined as one cycle, and while performing the above charge/discharge for 100 cycles, the capacity retention rate (life retention rate) compared to the initial charge capacity at room temperature was measured.

**[Table 1]**

| | | Electrolyte composition | Lithium salt (M) | Additive (parts by weight) | Solvent (volume ratio) | Life retention rate (%) |
|---|---|---|---|---|---|---|
| E X A M P L E | C1 | Example B1 | LiN(CF₃SO₂)₂ (1.0) | LiNO₃ (1) | 1,2-dimethoxyethane (50) | 93.4% |
| | | | | Compound of Example A1 (2) | 1,3-dioxolane (50) | |
| | C2 | Example B2 | LiN(CF₃SO₂)₂ (4.2) | LiNO₃ (4.5) | 1,2-dimethoxyethane (50) | 90.3 % |
| | | | | Compound of Example A2 (4.4) | 1,3-dioxolane (50) | |
| | C3 | Example B3 | LiN(SFO₂)₂ (1.0) | CH₃NO₃ (1) | 1,2-dimethoxyethane (50) | 92.9 % |
| | | | | Compound of Example A3 (4.3) | 1,3-dioxolane (50) | |
| | C4 | Example B4 | LiPF₆ (0.4) | LiNO₂ (0.5) | 1,2-dimethoxyethane (50) | 90.6 % |
| | | | | Compound of Example A1 (0.7) | 1,3-dioxolane (50) | |
| | C5 | Example B5 | LiN(CF₃SO₂)₂ (1.0) | LiNO₃ (1) | 1,2-dimethoxyethane (100) | 91.3 % |
| | | | | Compound of Example A1 (2) | | |
| | C6 | Example B6 | LiN(CF₃SO₂)₂ (1.0) | LiNO₃ (1) | tetraethyleneglycol dimethyl ether (25) | 92.2 % |
| | | | | Compound of Example A1 (2) | 1,3-dioxolane (75) | |
| | C7 | Example B7 | LiN(CF₃SO₂)₂ (1.0) | LiNO₃ (1) | 1,2-dimethoxyethane (50) | 93.1 % |
| | | | | Compound of Example A4 (2) | 1,3-dioxolane (50) | |
| | C8 | Example B8 | LiN(CF₃SO₂)₂ (1.0) | LiNO₃ (1) | 1,2-dimethoxyethane (50) | 94.8 % |
| | | | | Compound of Example A5 (2) | 1,3-dioxolane (50) | |
| | C9 | Example B9 | LiN(CF₃SO₂)₂ (1.0) | LiNO₃ (1) | 1,2-dimethoxyethane (50) | 90.8 % |
| | | | | Compound of Example A6 (2) | 1,3-dioxolane (50) | |
| | C10 | Example B10 | LiN(CF₃SO₂)₂ (1.0) | LiNO₃ (1) | 1,2-dimethoxyethane (50) | 92.1 % |
| | | | | Compound of Example A7 (2) | 1,3-dioxolane (50) | |
| C O M P. E X A M P L E | C1 | Comparative Example B1 | LiN(CF₃SO₂)₂ (1.0) | LiNO₃ (1) | 1,2-dimethoxyethane (50) | 64.2 % |
| | | | | | 1,3-dioxolane (50) | |
| | C2 | Comparative Example B2 | LiN(SFO₂)₂ (1.0) | LiNO₃ (0.04) | 1,2-dimethoxyethane (50) | 63.4 % |
| | | | | Compound of Example A1 (0.05) | 1,3-dioxolane (50) | |
| | C3 | Comparative Example B3 | LiN(CF₃SO₂)₂ (1.0) | LiNO₃ (5.5) | 1,2-dimethoxyethane (50) | 51.3 % |
| | | | | Compound of Example A1 (4.6) | 1,3-dioxolane (50) | |
| | C4 | Comparative Example B4 | LiN(CF₃SO₂)₂ (1.0) | LiNO₃ (0.04) | 1,2-dimethoxyethane (50) | 60.2 % |
| | | | | Compound of Example A4 (0.05) | 1,3-dioxolane (50) | |
| | C5 | Comparative Example B5 | LiN(CF₃SO₂)₂ (1.0) | LiNO₃ (5.5) | 1,2-dimethoxyethane (50) | 55.8 % |
| | | | | Compound of Example A5 (4.6) | 1,3-dioxolane (50) | |
| | C6 | Comparative Example B6 | LiN(CF₃SO₂)₂ (1.0) | LiNO₃ (0.04) | 1,2-dimethoxyethane (50) | 68.5 % |
| | | | | Compound of Example A6 (0.05) | 1,3-dioxolane (50) | |
| | C7 | Comparative Example B7 | LiN(CF₃SO₂)₂ (1.0) | LiNO₃ (5.5) | 1,2-dimethoxyethane (50) | 56.2% |
| | | | | Compound of Example A7 (4.6) | 1,3-dioxolane (50) | |

As shown in Table 1 above, in the case of secondary batteries using the electrolyte compositions of Examples B 1 to B 10 according to the present invention, since the additive containing the compound of formula 1 of the present invention was comprised in an amount specified in the present invention, the life retention rate (capacity retention rate) was very high as 90.0% or higher, regardless of the type of lithium salt.

However, in the case of secondary battery using the electrolyte composition of Comparative Example B 1 not containing the compound of formula 1 of the present invention, the life retention rate was poor as 64.2%; in the case of secondary batteries using the electrolyte compositions of Comparative Examples B2, B4 and B6 in which the additive containing the compound of formula 1 (concretely, compound of any of formulas 2 to 4) of the present invention was used in an amount less than the amount specified in the present invention, the life retention rate was poor as lower than 70%; and in the case of secondary batteries using the electrolyte compositions of Comparative Examples B3, B5 and B7 in which the additive containing the compound of formula 1 (concretely, compound of any of formulas 2 to 4) of the present invention was used in an amount greater than the amount specified in the present invention, the life retention rate was very poor as lower than 60%.

## Claims

1. A compound represented by formula 1: 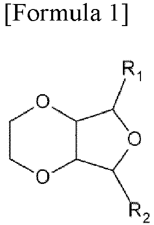 wherein R₁ and R₂ are each independently selected from the group consisting of hydrogen atom, halogen atom, substituted or unsubstituted C1-C30 alkyl group, substituted or unsubstituted C2-C30 alkenyl group, substituted or unsubstituted C2-C30 alkynyl group, substituted or unsubstituted C1-C30 alkoxy group, substituted or unsubstituted C3-C30 cycloalkyl group, substituted or unsubstituted C1-C30 alkylene-O-C1-C30 alkyl group, substituted or unsubstituted C3-C30 heterocycloalkyl group, substituted or unsubstituted C6-C60 aryl group, substituted or unsubstituted C1-C60 heteroaryl group, substituted or unsubstituted C7-C60 aralkyl group, substituted or unsubstituted C6-C60 aryloxy group, and substituted or unsubstituted C6-C60 arylthio group, provided that at least one of R₁ and R₂ is not hydrogen atom.

2. The compound of claim 1, wherein the compound represented by formula 1 is selected from the group consisting of compounds represented by formulas 2 to 4: 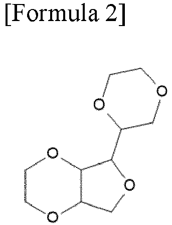 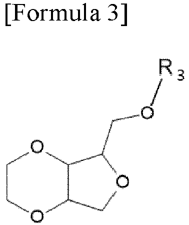 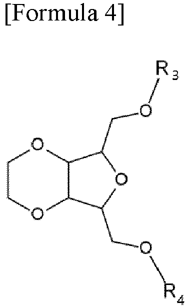 wherein R₃ and R₄ are each independently substituted or unsubstituted C1-C30 alkyl group.

3. The compound of claim 1, wherein
the compound represented by formula 2 is selected from the group consisting of compounds represented by formulas 2-1 to 2-3;
the compound represented by formula 3 is a compound represented by formula 3-1 or 3-2; and
the compound represented by formula 4 is a compound represented by formula 4-1 or 4-2:

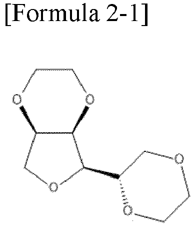

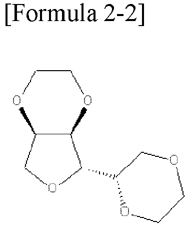

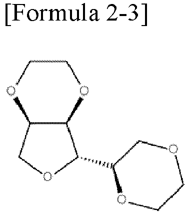

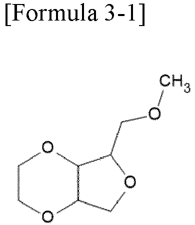

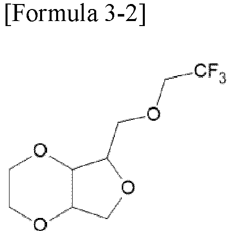

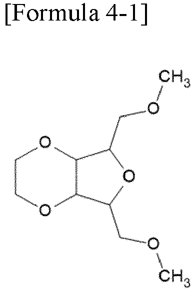

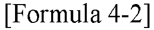

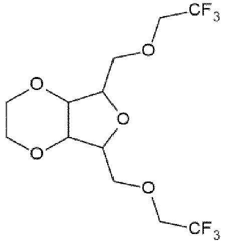

4. A method for preparing a compound represented by formula 2, the method comprising a step of heating dianhydrosugar hexitol-ethylene glycol, which is a compound in which ethylene glycol is added to the hydroxy groups at both ends of dianhydrosugar hexitol, in the presence of an acid catalyst:

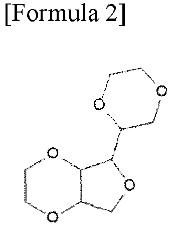

5. The method of claim 4, wherein the dianhydrosugar hexitol-ethylene glycol is that prepared by addition reaction of dianhydrosugar hexitol and ethylene oxide.

6. The method of claim 5, wherein the addition reaction of the dianhydrosugar hexitol and ethylene oxide is conducted with using 2 moles or more of ethylene oxide, based on 1 mole of dianhydrosugar hexitol.

7. The method of claim 4, wherein the acid catalyst is selected from sulfuric acid, hydrochloric acid, hydrobromic acid, phosphoric acid, methanesulfonic acid, benzenesulfonic acid, para-toluenesulfonic acid, or a combination thereof.

8. A method for preparing a compound represented by formula 3 or 4, the method comprising a step of heating a compound represented by formula A or B, respectively, and an alkylating agent in the presence of a base catalyst:

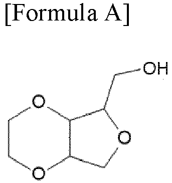

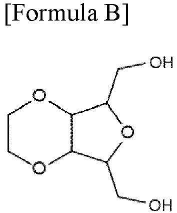

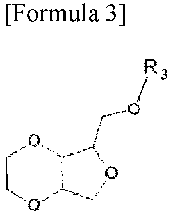

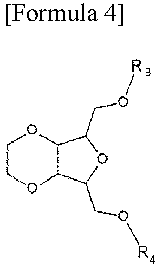

9. The method of claim 8, wherein the alkylating agent is selected from alkyl halide of formula R-X [wherein R is a substituted or unsubstituted C1-C30 alkyl group, and X is a halogen atom], aromatic sulfonate having substituted or unsubstituted C1-C30 alkyl group, or combination thereof.

10. The method of claim 8, wherein the base catalyst is selected from inorganic base catalyst, organic base catalyst, or combination thereof.

11. An electrolyte additive comprising the compound represented by formula 1: 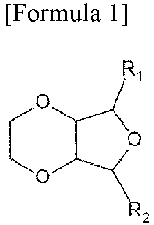 wherein R₁ and R₂ are each independently selected from the group consisting of hydrogen atom, halogen atom, substituted or unsubstituted C1-C30 alkyl group, substituted or unsubstituted C2-C30 alkenyl group, substituted or unsubstituted C2-C30 alkynyl group, substituted or unsubstituted C1-C30 alkoxy group, substituted or unsubstituted C3-C30 cycloalkyl group, substituted or unsubstituted C1-C30 alkylene-O-C1-C30 alkyl group, substituted or unsubstituted C3-C30 heterocycloalkyl group, substituted or unsubstituted C6-C60 aryl group, substituted or unsubstituted C1-C60 heteroaryl group, substituted or unsubstituted C7-C60 aralkyl group, substituted or unsubstituted C6-C60 aryloxy group, and substituted or unsubstituted C6-C60 arylthio group, provided that at least one of R₁ and R₂ is not hydrogen atom.

12. The electrolyte additive of claim 11, further comprising nitric (or nitrous) acid-based compound.

13. A non-aqueous electrolyte composition comprising: a non-aqueous electrolyte solvent; lithium salt; and the electrolyte additive of claim 11 or 12, wherein the amount of the electrolyte additive is from 0.1 to 10 parts by weight, based on total 100 parts by weight of the electrolyte composition.

14. A secondary battery comprising: a cathode; an anode; a separation membrane; and the non-aqueous electrolyte composition of claim 13.

15. The secondary battery of claim 14, which is a lithium-sulfur secondary battery.
